(19) Europäisches Patentamt
European Patent Office
Office européen des brevets

(11) Publication number: 0 486 536 B1

# (12) EUROPEAN PATENT SPECIFICATION

(45) Date of publication of patent specification: 26.10.94

(51) Int. Cl.5: A61K 31/70

(21) Application number: 90911686.5

(22) Date of filing: 03.08.90

(86) International application number:
PCT/US90/04270

(87) International publication number:
WO 91/02521 (07.03.91 91/06)

## (54) ANTIVIRAL COMPOSITIONS CONTAINING SULFOOUINOVOSYL GLYCEROL DERIVATIVES AND ANALOGS THEREOF.

(30) Priority: 15.08.89 US 393780

(43) Date of publication of application:
27.05.92 Bulletin 92/22

(45) Publication of the grant of the patent:
26.10.94 Bulletin 94/43

(84) Designated Contracting States:
AT BE CH DE DK ES FR GB IT LI LU NL SE

(56) References cited:
WO-A-89/03684

JOURNAL OF THE NATIONAL CANCER INSTITUTE, vol. 81, no. 16, August 16, 1989, pages 1254-1258; K.R. GUSTAFSON et al.: "AIDS-antiviral sulfolipids from cyanobacteria (Blue-green algae)"

(73) Proprietor: THE UNITED STATES OF AMERICA as represented by the Secretary United States Department of Commerce
National Technical Information Service,
Office of Government Inventions and Patents,
5285 Port Royal Road
Springfield, Virginia 22161 (US)

(72) Inventor: BOYD, Michael, R.
5217 Fairgreene Way
Ijamsville, MD 21754 (US)
Inventor: CARDELLINA, John, H., II
NCI/FCRDC
Building 1052
Frederick, MD 21701 (US)
Inventor: SNADER, Kenneth, M.
2503 Baltimore Road
Rockville, MD 20853 (US)
Inventor: GUSTAFON, Kirk, R.
3508 Edwin Street
Wheaton, MD 20902 (US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

VESTN. MOSK. UNIV. SER. VIBIOL. POCHVOVED., vol. 27, no. 4, 1972, pages 108-110; G.E. JUKOVA et al.: "Inactivation of some RNA-contained viruses with green and blue-green algae"

Inventor: **PATTERSON, Gregory, M., L.**
**2740 Kuilei Street, 905**
**Honolulu, HI 96826 (US)**
Inventor: **MCMAHON, James, B.**
**529 Ellrose Court**
**Frederick, MD 21701 (US)**
Inventor: **WEISLOW, Owen, S.**
**11909 Winstead Lane**
**Reston, VA 22094 (US)**
Inventor: **SHOEMAKER, Robert, H.**
**18526 Grouse Lane**
**Gaithersburg, MD 20879 (US)**
Inventor: **PAULL, Kenneth, D.**
**4 Relda Court**
**Gaithersburg, MD 20878 (US)**

(74) Representative: **Davies, Jonathan Mark**
**Reddie & Grose**
**16 Theobalds Road**
**London WC1X 8PL (GB)**

## Description

### BACKGROUND OF THE INVENTION

### Field of the Invention

The present invention relates to compositions containing sulfonic acid-containing glycolipids which exhibit antiviral activity.
More specifically, the present invention relates to compositions containing sulfoquinovosyl glycerol derivatives and analogs thereof which exhibit advantageous pharmacological, toxicological and antiviral properties, such as for example inhibition of the cytopathic effects of the human immunodeficiency virus (HIV) which is implicated as a causitive agent of AIDS (Acquired Immune Deficiency Syndrome).

### Description of Related Art

AZT is a known clinically active agent currently used in the therapy of AIDS. However, while extremely useful in antiviral therapy, AZT is limited by toxicity and an insufficient therapeutix index to make it adequate for therapy. Thus, new classes of antiviral agents to be used alone or in combination with AZT and other agents are urgently needed for effective antiviral therapy.

Although sulfonic acid-containing lipids have been previously described in the chemical literature, their association with antiviral activity has not heretofore been discovered. Sulfonic acid-containing lipids have previously been described in the chemical literature. A.A. Benson et al., Proc. Natl. Acad. Sci. U.S.A., 45, p. 1582 (1959); A.A. Benson, Adv. Lipid Res., 1, P 387 (1963). Members of this structural class are commonly referred to as sulfoquinovosyl diacylglycerols. These lipids occur as structural components of chloroplast membranes and reportedly are distributed widely in higher plants, algae and photosynthetic microorganisms. J.B. Mudd et al. in The Biochemistry of Plants, P.K. Stumpf, Ed. (Academic Press, New York, 1987), Vol. 9, p. 275; J.L. Harwood, ibid., (1980), Vol. 4, p. 301. Sulfolipids are ubiquitous in common food plants and are routinely ingested as part of the human diet, however, metabolic studies indicate that in mammals, sulfoquinovosyl diacylgiycerols are not absorbed intact but are extensively degraded in the gastrointestinal tract. S.D. Gupta et al., Arch. Biochem. Biophys., 259, p. 510 (1987). The modified polysaccharide dextran sulfate (H. Mitsua et al., Science, 240 p. 646 (1988)) and sulfated carageenan (H. Nakashima et al, Antimicrob. Agents Chemother. 31, p. 1524 (1987)) reportedly inhibit HIV *in vitro*, but these are sulfate esters, not sulfonic acids.

### SUMMARY OF THE INVENTION

Therefore, it is an object of the present invention to provide compositions containing sulfoquinovosyl glycerol derivatives and analogs thereof which exhibit antiviral activity and a pharmaceutically acceptable excipient.

The present invention provides a composition containing sulfoqulnovosyl glycerol derivatives of the following formula:

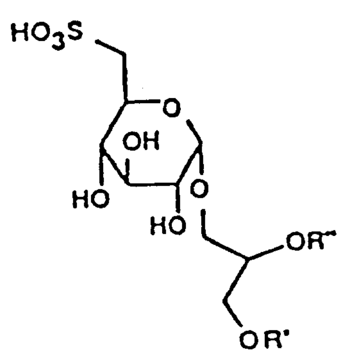

Formula(I)

wherein R' is a straight chain unsaturated or saturated fatty acid ester and R" is a straight chain saturated fatty acid ester; and a pharmaceutically acceptable excipient; for use in the treatment of viral infections.

The invention also provides for the use of a sulfoquinovosyl glycerol compound of the formula

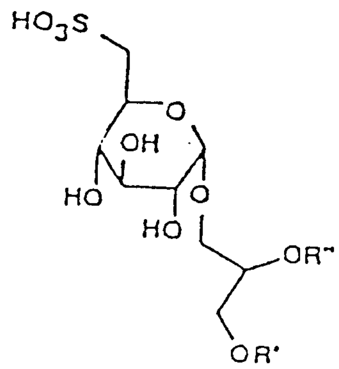

wherein R' is a straight chain saturated or unsaturated fatty acid ester and R" is a straight chain saturated fatty acid ester in the preparation of a medicament for the treatment of viral infections of a host.

## BRIEF DESCRIPTION OF THE DRAWINGS

The invention is further illustrated in the accompanying drawings wherein:

Figures 1(A) and 1(B) illustrate protection afforded CEM cells by fractions obtained during the purification process;

Figure 1(C) illustrates the results of a CEM treatment with purified sulfolipid **1**;

Figure 2(A) illustrates the anti-HIV activity of sulfolipid **4** observed in the CEM, C3-44, LDV-7, and MT-2 cell lines;

Figure 2(B) illustrates the correlation of increased formazan production from XTT, with a decrease in infectious virus as measured by syncytia forming units (SFU) and with a decrease in the HIV core protein p24; and

Figure 3 illustrates the results of CEM treatment with sulfolipid **5**.

## DETAILED DESCRIPTION OF THE INVENTION

As part of the U.S. National Cancer Institute (NCI) program to discover new antitumor and antiviral agents from natural sources (M.R. Boyd, in AIDS Etiology, Diagnosis, Treatment and Prevention, V.T. Devita, Jr. et al., Eds. (J.B. Lippincott, Philadelphia 1988), p. 305), extracts from a representative series of cyanobacteria ("bluegreen algae") were screened for anti-HIV properties. Cellular extracts from cultured Lyngbya lagerheimii and Phormidium tenue protected human T-lymphoblastoid cells from the cytopathic effect of HIV infection. Using an in vitro anti-HIV assay to guide fractionation, a series of sulfolipids was isolated by a combination of gel permeation and reversed phase chromatographies as described in detail with regard to Example 1 below. These isolated sulfolipids include compounds of the following formula:

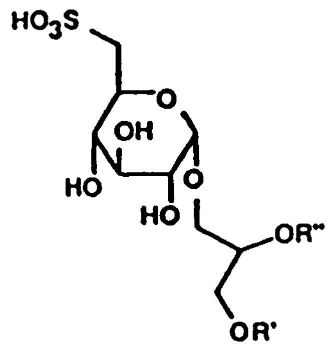

**Formula (I)**

wherein R' is a straight chain unsaturated fatty acid ester and R'' is a straight chain saturated fatty acid ester. The structures of four isolated compounds, i.e. compounds **1-5**, were found to include fatty acid esters having the following carbon atom to double bond ratios:

| Compound No. | R' | R'' |
|---|---|---|
| **1** | 18:3 | 16:0 |
| **2** | 18:2 | 16:0 |
| **3** | 18:1 | 16:0 |
| **4** | 16:1 | 16:0 |
| **5** | 18:0 | 16:0 |

Compound **5** was produced by the catalytic reduction of sulfolipid **1** to its fully saturated analog. The hydrogenation was effected by stirring an ethanolic solution of compound **1** with 10% palladium on charcoal under one atmosphere of hydrogen for 10 hours.

Compound **1** was found to have the following structure:

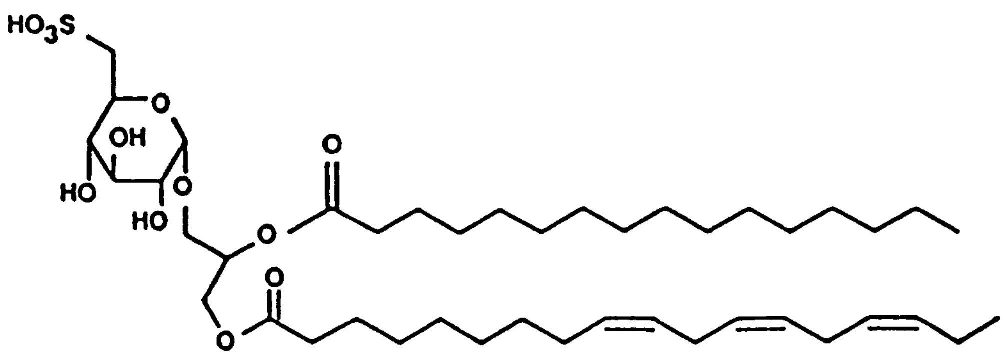

**Formula (II)**

The structures of compounds **1-5** were assigned by interpretation of their spectral characteristics, particularly their $^1H$ and $^{13}C$ NMR and mass spectral features. The spectral features of sulfolipids **1-5** were recorded as follows:

Compound **1** $[\alpha]_D$ = +55.5° (c 0.98, MeOH), PIFAB MS: m/z 839 ($M+Na^+$), 817 ($M+H^+$); NIFAB MS: m/z 815 ($M-H^-$), calculated 816 for $C_{43}N_{76}O_{12}S$; IR ($CCl_4$): 3600-3200, 2915, 2850, 1730 $cm^{-1}$; $^1H$ NMR (200 MHz, $CD_3OD$):$\delta$ 0.88 (3H, br t), 0.96 (3H, t, J = 7.6), 1.26 (32H, br), 1.59 (4H, m), 2.07 (4H, m), 2.31 (4H, m), 2.79 (4H, m), 2.89 (1H, dd, J = 14.4, 9.2), 3.06 (1H, dd, J = 9.8, 9.0), 3.30 (1H), 3.32 (1H), 3.55

(1H, dd, J, = 11.0, 6.4), 3.61 (1H, dd, J = 9.6, 9.0), 4.05 (1H), 4.09 (1H), 4.17 (1H, dd, J = 11.8, 7.0), 4.49 (1H, dd, J = 11.8, 3.2), 4.74 (1H, d, J = 3.8), 5.25 (1H, m), 5.32 (6H, m); $^{13}C$ NMR (200 MHz, $CD_3OD$): δ 14.5 (q), 14.7 (q), 21.5 (t), 23.8 (t), 26.1 through 30.8 (19C, all t), 33.1 (t), 35.0 (t), 35.3 (t), 54.4 (t), 64.3 (t), 67.2 (t), 69.9 (d), 71.8 (d), 73.5 (d), 75.0 (d), 75.1 (d), 100.1 (d), 128.2 (d), 128.8 (d), 129.2 (2C, each d), 131.0 (d), 132.7 (d), 174.8 (s), 175.0 (s);

Compound **2** $[\alpha]_D$ = + 62.8° (c 0.83, MeOH); NIFAB MS: m/z 817 (M-H⁻), calculated 818 for $C_{43}H_{78}O_{12}S$; $^1H$ NMR ($CD_3OD$) was identical to that of compound **1** except for the following: δ 0.88 (6H, br t), 2.79 (2H, m), 5.32 (4H, m); $^{13}C$ NMR ($CD_3OD$) was virtually identical to that of compound 1 except there were only four resonances in the olefinic region: δ 128.9 (d), 129.0 (d), 130.8 (d), 130.9 (d);

Compound **3** NIFAB MS: m/z 819 (M-H⁻), calculated 820 for $C_{43}H_{80}O_{12}S$; $^1H$ NMR ($CD_3OD$) was virtually identical to that of compound **1** except for the lack of a signal at δ 2.79 and the reduced integral of the signal at δ 5.32 (2H, m);

Compound **4** NIFAB MS: m/z 791.5033 (M-H⁻), calculated 791.4979 for $C_{41}H_{75}O_{12}S$; $^1H$ NMR ($CD_3OD$) was virtually identical to that of compound **3**.

Compound **5** NIFAB MS: m/z 821 (M-H⁻) was calculated 822 for $C_{43}H_{82}O_{12}S$.

The molecular formulae were established by fast-atom bombardment mass spectrometry. Characteristic $^1H$ NMR resonances, in combination with appropriate $^{13}C$ NMR signals, indicated a series of diacylglycerol glycosides that differed only in their fatty acid acyl constituents. Proton decoupling and COSY NMR (4) experiments demonstrated that the sugar moiety was an α -linked hexapyranose with substitution pattern and relative stereochemistry similar to glucose. Molecular formula consideration and the compounds' retention by anion exchange resin suggested the presence of a sulfonic acid residue. Characteristic $^{13}C$ NMR (54.4 t) and $^1H$ NMR (2.89, 1H, dd, J = 14.4, 9.2 and 3.30, 1H) resonances supported placement of a sulfonic acid on the 6' carbon of the sugar. Mass spectral fragments due to cleavage between $C_1$ and $C_2$ of the glycerol subunit allowed the positional assignment of the acyl groups (5). The absolute stereochemistry of the glycerol was established as "D" by deacylation of compound 1 to the sulfoquinovosyl glycerol and comparison of the optical rotation of its cyclohexylammonium salt $[\alpha]_D$ = +60.5° to the literature value $[\alpha]_D$ = +74.5° as reported by Lepage et al., J. Am. Chem. Soc., Vol. 83, P. 157 (1961). Compound 1 was hydrolyzed with 0.2 N methanolic KOH at 37°C for 90 minutes. The resulting sulfoquinovosyl glycerol salt was converted to the free acid on AG 50W-X2 (hydrogen form) ion exchange resin and treated with a slight excess of cyclohexylamine to form the cyclohexylammonium salt. Linolenic acid, identical to an authentic sample by $^{13}C$ NMR and mass spectral analysis, was also recovered from the hydrolysate of compound **1**. The extract of L. lagerheimii contained compounds **1** and **2**, while P. tenue provided **3** and **4**. Additional sulfolipids were present in each extract, but they occurred as inseparable mixtures.

The combined sulfolipid fraction constituted approximately 10% by weight of the organic extractables from the cyanobacterial cells. Additional cultured cyanobacterial extracts which inhibited HIV included Phormidium cebennse, Oscillatoria raciborskii. Scytonema burmanicum, Calothrix elenkinii, and Anabaena variabilis. All tested positive for the presence of sulfolipids. All cultures were grown and extracted at the University of Hawaii. Unialgal, non-axenic strains of L. lagerheimii (strain DN-7-1) and P. tenue (strain CN-2-1), obtained from collections in Hawaii and Palau, respectively, were transferred after six passes to 25 liter culture bottles containing a modification of Allen's medium #3 (M.B. Allen, Arch. Mikro. Vol. 17, p. 34 (1952)) at an initial pH of 7.00. Aeration was performed at 0.2 vvm (liters gas per liter liquid per minute) with a sterile stream of air supplemented with 0.5% carbon dioxide. Illumination was provided by fluorescent tubes with an intensity of 300 uE/$m^2$/sec. Cultures were continued until they reached early stationary growth phase (idiophase), which typically occurred between 24 and 28 days. The cellular mass was harvested by filtration and extracted with 20 volumes of 1:1 methanol-dichloromethane. Thus, 16.5 g. of wet cells from L. lagerheimii yielded 1.87 g. of extract and 10.57 g. of wet cells from P. tenue yielded 1.02 g. of extract. Aqueous extracts of the cell mass failed to show any significant HIV-inhibitory activity.

The purified sulfolipids were evaluated for HIV-inhibitory effects using a newly developed, tetrazolium-based in vitro assay which has recently been described in detail (O.S. Weslow, et al. Journal of the National Cancer Institute 81:577-586, 1989). The assay detects drug-induced suppression in virus cytopathic effects by measuring the metabolic reduction in surviving cells of a tetrazolium salt ("XTT"), in the presence of an electron transfer reagent, N-methylphenazonium methosulfate ("PMS"), to a soluble formazan. The colored formazan product can be measured by conventional colorimetric techniques.

Compounds **1-4** were evaluated using the MT-2, CEM, LDV-7 and C3-44 human T-lymphoblastoid cell lines. Compound 5 was tested using CEM cells. HIV infection was induced by the addition of cell free virions or cocultivation with chronically infected H-9 cells. The HIV inhibitory activity of the sulfolipids was generally consistent within a given cell line, but there was substantial variation in the degree of protection among the different cell lines (Fig. 2(A)). The protective effects of the sulfolipids were observed over a

broad concentration range (approximately 1-100 μg/ml), depending upon the target cell line and the mode of infection. All of the sulfolipids tested had similar levels of activity, suggesting that acyl chain length and degree of unsaturation, at least over the small variation examined, do not critically affect potency. Using the CEM line, the increase in formazan production was correlated with a corresponding decrease in infectious virions as measured by HIV-induced syncytia formation and p24 antigen synthesis (Fig. 2(B) below). Structurally related acyl glycerols, complex lipids, detergents and simple sulfonic acid derivatives did not exhibit any protection against HIV infection in our assay. Compounds negative in the tetrazolium anti-HIV assay included dioleoylglycerol, monolinolenoylglycerol, phosphatidylcholine, galactocerebrosides, octylglucopyranoside, NP-40, heptanesulfonic acid, p-toluenesulfonic acid and N-morpholinosulfonic acid.

EXAMPLE 1

Anti-HIV activity guided fractionation and purification of sulfolipids.

Figures 1(A) and 1(B) illustrate protection (eq. 1) afforded CEM cells (at 250 μg/ml) by fractions obtained during the purification process. Figure 1(C) illustrates the results of CEM treatment with purified sulfolipid 1. Formazan production by the uninfected, drug treated cells (-◆-), virus infected, drug treated cells (-□-) and virus infected, untreated cells (-■-) is expressed as a percentage of the formazan produced by uninfected, untreated cells (eq. 2). Fractions and pure compounds were bioassayed using a tetrazolium-based microculture procedure, as follows: Dilutions of test compounds (100 μl) were mixed with target cells in 96 well trays (100 μl, 10,000 cells/well) and infection was induced by cocultivation with H9 cells (400 cells/well) chronically infected with HIV-1 (IIIb variant). Following a seven day incubation at 37°C in air-$CO_2$, a mixture of the tetrazolium salt XTT and PMS, was added and formazan production was allowed to proceed for 4 hours. Quantitative analysis was accomplished by measuring the optical density (O.D.) at 450 nm (test wavelength) and 650 nm (reference wavelength). Data are expressed either as percent protection or percent of untreated control formazan production from the formulae:

$$\%\ \text{protection} = \frac{\text{test O.D.} - \text{virus control O.D.}}{\text{cell control O.D.} - \text{virus control O.D.}} \times 100 \quad \text{[eq.1]}$$

$$\%\ \text{of untreated} = \frac{\text{test O.D.}}{\text{cell control O.D.}} \times 100 \quad \text{[eq.2]}$$

EXAMPLE 2

Figure 2(A) graphically illustrates the anti-HIV activity of sulfolipid compound **4** observed in the CEM (-□-), C3-44 (-◆-), LDV-7 (-◇-), and MT-2 (-□-) cell lines. The assay was performed as described in Example 1 above except that the cells were infected with cell free virus (RF variant) using an MOI of .01 (CEM), .001 (C3-44), .01 (LDV-7), or .001 (MT-2).

Figure 2(B) illustrates the correlation of increased formazan production from XTT (-□-), with a decrease in infectious virus as measured by syncytia forming units (SFU) (-◆-) and with a decrease in the HIV core protein p24 (-◇-). Supernatant fluids from the test wells (50 μl) were harvested just prior to the time of tetrazolium addition. Aliquots were examined immediately using the syncytium assay described by Nara et al. (AIDS Res. Hum. Retroviruses, vol. 3, p. 283 (1987)): supernatants from test plates (40 μl) were examined in CEM cell monolayers at multiple dilutions to obtain countable levels of syncytial forming units (SFU) (50-200 SFU/well) in 4 days. The remaining 10 μl were diluted 1:100 with Triton X-100 and stored frozen at -70°C until required for the p24 ELISA assay (Dupont). 200 μl aliquots of the Triton-treated samples were added to duplicate microtiter wells previously coated with rabbit polyclonal anti-HIV p24 serum and incubated at room temperature overnight. After washing and blotting, 100 μl of biotinylated polyclonal anti-HIV p24 was added to each of the appropriate wells and the plates were reincubated at 37°C for 60 minutes. A solution of Strepavidin-horseradish peroxidase was added after initial washing and blotting. The plates were mixed and reincubated for 15 minutes at room temperature and after washington and blotting the ODP substrate was added in 100 μl aliquots. Color was allowed to develop in the dark room at room temperature for 30 minutes and the reaction was halted by the addition of 4N $H_2SO_4$. Optical density was measured at 490 nm and the concentration of p24 was determined by comparison with a

standard curve of known p24 samples.

EXAMPLE 3

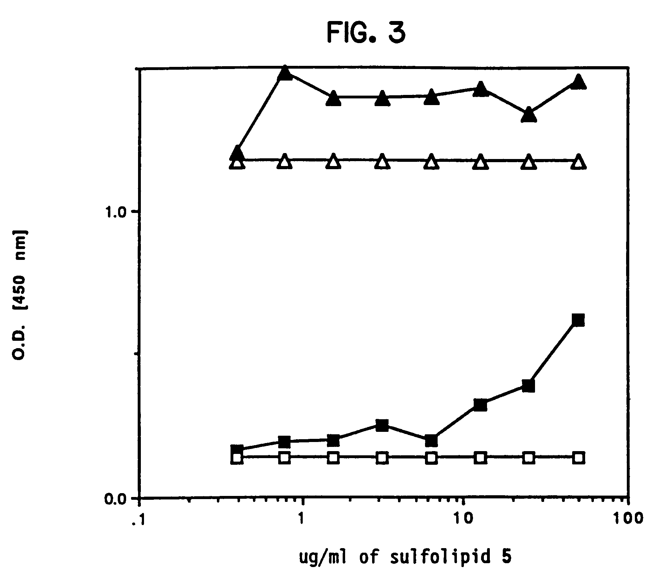

Figure 3 illustrates the results of CEM treatment with sulfolipid **5** in the XTT tetrazolium assay. Optical density (O.D.) readings at 450 nm are due to formazan production by uninfected, untreated cells (-△-), virus infected, untreated cells (-□-), virus infected, sulfolipid treated cells (-■-) and uninfected, sulfolipid treated cells (-▲-). The assay was performed as described in Example 2.

The sulfoquinovosyl glycerol derivatives employed in the present invention may be made into pharmaceutical compositions by combination with appropriate pharmaceutically acceptable carriers or diluents, and may be formulated into preparations in solid, semi-solid, liquid or gaseous forms such as tablets, capsules, powders, granules, ointments, solutions, suppositories, injections, inhalants, and aerosols in the usual ways for their respective route of administration. The following methods and excipients are merely exemplary.

In pharmaceutical dosage forms, the sulfoquinovosyl glycerol derivatives employed in the present invention may be used in the form of their pharmaceutically acceptable salts, and also may be used alone or in appropriate association, as well as in combination with other pharmaceutically active compounds.

In the case of oral preparations, the sulfoquinovosyl glycerol derivatives may be used alone or in combination with appropriate additives to make tablets, powders, granules or capsules, e.g. with conventional additives such as lactose, mannitol, corn starch or potato starch; with binders such as crystalline cellulose, cellulose derivatives, acacia, corn starch or gelatins; with disintegrators such as corn starch, potato starch or sodium carboxymethylcellulose; with lubricants such as talc or magnesium stearate; and if desired, with diluents, buffering agents, moistening agents, preservatives and flavoring agents.

Furthermore, the sulfoquinovosyl glycerol derivatives employed in the present invention may be made into suppositories by mixing with a variety of bases such as emulsifying bases or water-soluble bases.

The sulfoquinovosyl glycerol derivatives employed in the present invention may be formulated into preparations for injections by dissolving, suspending or emulsifying them in an aqueous or non-aqueous solvent, such as vegetable oil, synthetic aliphatic acid glycerides, esters of higher aliphatic acids or propylene glycol; and if desired, with conventional additives such as solubilizers, isotonic agents, suspending agents, emulsifying agents, stabilizers and preservatives.

In the cases of inhalations or aerosol preparations, the sulfoquinovosyl glycerol derivatives employed in the invention in the form of a liquid or minute powder may be filled up in an aerosol container with gas or liquid spraying agents, and if desired, together with conventional adjuvants such as humidifying agents. They may also be formulated as pharmaceuticals for non-pressured preparations such as in a nebulizer or an atomizer.

The amount of the sulfoquinovosyl glycerol derivatives employed in the present invention to be used varies according to the degree of the infection encountered, and the stages of the disease. A suitable dosage is that which will result in concentration of the sulfoquinovosyl glycerol derivative (in blood and/or tissues harboring virus) which are known to inhibit the virus, e.g. about 1-250 $\mu$g/ml. The preferred dosage is that amount sufficient to render a host asymptomatic to the particular viral infection. The dose may vary when the compounds are used prophylactically.

Unit dosage forms for oral administration such as syrups, elixirs, and suspensions wherein each dosage unit, e.g., teaspoonful, tablespoonful, contains a predetermined amount of the sulfoquinovosyl glycerol derivatives employed in the present invention can be by a pharmaceutically acceptable carrier, such as Sterile Water for Injection, USP, or by normal saline.

The sulfoquinovosyl glycerol derivatives employed in the present invention can be administered rectally via a suppository. The suppository can include vehicles such as cocoa butter, carbowaxes and polyethylene glycols, which melt at body temperature, yet are solidified at room temperature.

The sulfoquinovosyl glycerol derivatives employed in the present invention can be utilized in aerosol formulation to be administered via inhalation. The sulfoquinovosyl glycerol derivatives employed in the present invention can be formulated into pressurized acceptable propellants such as dichlorodifluoromethane, propane, nitrogen.

The term "unit dosage form" as used herein refers to physically discrete units suitable as unitary dosages for human and animal subjects, each unit containing a predetermined quantity of the sulfoquinovosyl glycerol derivatives calculated in an amount sufficient to produce the desired effect in association with a pharmaceutically acceptable, diluent, carrier or vehicle. The specifications for the novel unit dosage forms of the present invention depend on the particular compound employed and the effect to be achieved, and the pharmacodynamics associated with each compound in the host.

The pharmaceutically acceptable excipients, for example, vehicles, adjuvants, carriers or diluents are readily available to the public.

Any necessary adjustments in dose can be readily made to meet the severity of the infection and adjusted accordingly by the skilled practitioner.

## Claims

1. A composition which comprises sulfoquinovosyl glycerol compound of the formula

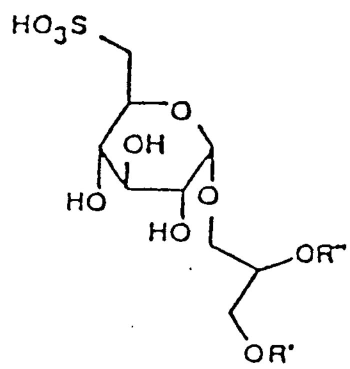

wherein R' is a straight chain saturated or unsaturated fatty acid ester and R'' is a straight chain saturated fatty acid ester; and a pharamaceutically acceptable excipient; for use in the treatment of viral infections.

2. The composition for the use as defined in claim 1, wherein said R'' fatty acid ester has a carbon atom to double bond ratio of 16:0 and said R' fatty acid ester has a carbon atom to double bond ratio selected from the group constisting of 18:3, 18:2, 18:1 and 16:1.

3. The composition for the use as defined in claim 2, wherein said sulfoquinovosyl glycerol compound is a compound of the following formula:

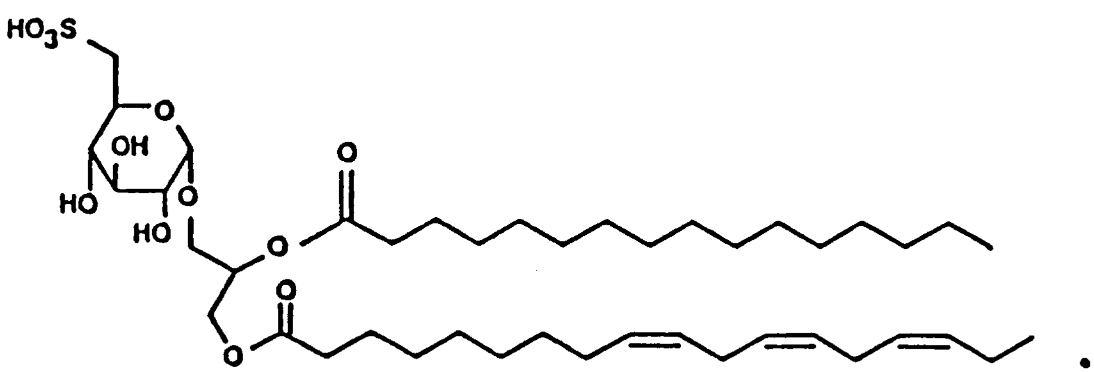

4. The composition for the use as defined in claim 2, wherein R' is 18:2.

5. The composition for the use as defined in claim 2, wherein R' is 18:1.

6. The composition for the use as defined in claim 2, wherein R' is 16:1.

7. The composition for the use as defined in claim 2, wherein R' is 18:0.

8. Use of a sulfoquinovosyl glycerol compound of the formula

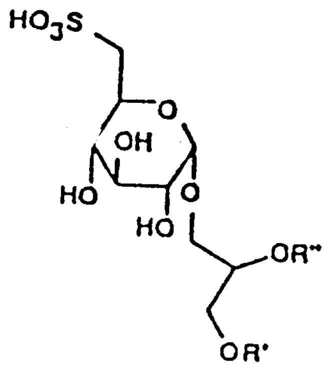

wherein R' is a straight chain saturated or unsaturated fatty acid ester and R" is a straight chain saturated fatty acid ester in the preparation of a medicament for the treatment of viral infections of a host.

9. Use according to claim 8 wherein said R" fatty acid ester has a carbon atom to double bond ratio of 16:0 and said R' fatty acid ester has a carbon atom to double bond ratio selected from the group consisting of 18:3, 18:2, 18:1 and 16:1.

10. Use according to claim 8 wherein said viral infection is caused by human immunodeficiency virus.

11. Use according to claim 9 wherein said sulfoquinovosyl glycerol compound is a compound of the following formula:

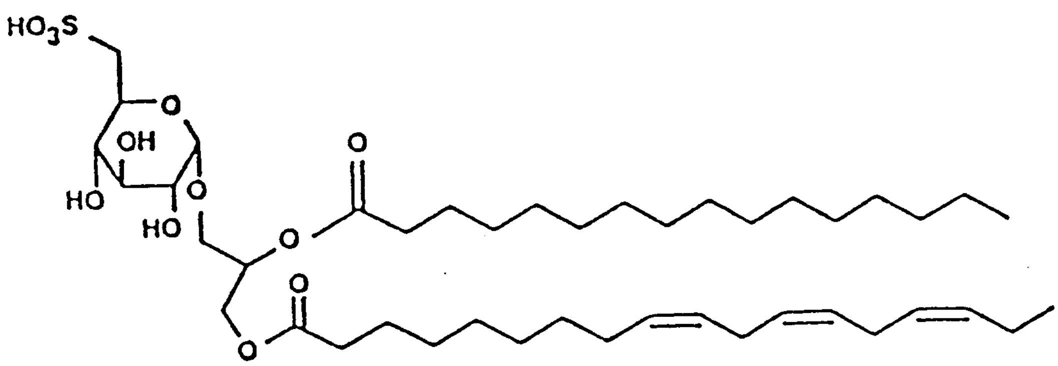

12. Use according to claim 9 wherein R' is 18:2.

13. Use according to claim 9 wherein R' is 18:1.

14. Use according to claim 9 wherein R' is 16:1.

15. Use according to claim 9 wherein R' is 18:0.

## Patentansprüche

1. Zusammensetzung, die Sulfoquinovosylglycerol-Verbindung der Formel

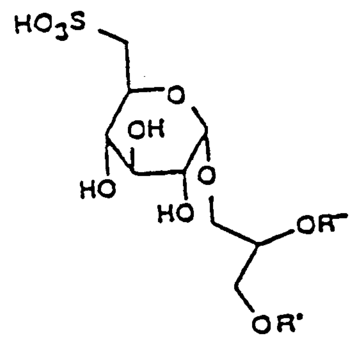

worin R' ein geradkettiger gesättigter oder ungesättigter Fettsäureester und R" ein geradkettiger gesättigter Fettsäureester ist, und ein pharmazeutisch annehmbares Exzipiens umfaßt, zur Verwendung bei der Behandlung von viralen Infektionen.

2. Die Zusammensetzung für die Verwendung, wie in Anspruch 1 definiert, worin besagter R"-Fettsäureester ein Kohlenstoffatom zu Doppelbindung-Verhältnis von 16:0 hat und der R'-Fettsäureester ein Kohlenstoffatom zu Doppelbindung-Verhältnis, ausgewählt aus der Gruppe bestehend aus 18:3, 18:2, 18:1 und 16:1, hat.

3. Die Zusammensetzung für die Verwendung wie in Anspruch 2 definiert, worin die Sulfoquinovosyglycerol-Verbindung eine Verbindung der folgenden Formel ist:

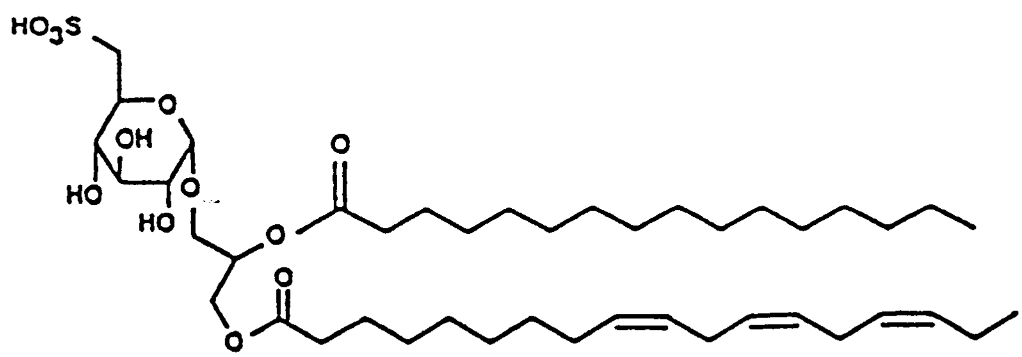

4. Die Zusammensetzung für die Verwendung wie in Anspruch 2 definiert, worin R' 18:2 ist.

5. Die Zusammensetzung für die Verwendung wie in Anspruch 2 definiert, worin R' 18:1 ist.

6. Die Zusammensetzung für die Verwendung wie in Anspruch 2 definiert, worin R' 16:1 ist.

7. Die Zusammensetzung für die Verwendung wie in Anspruch 2 definiert, worin R' 18:0 ist.

8. Verwendung einer Sulfoquinovosylglycerol-Verbindung der Formel

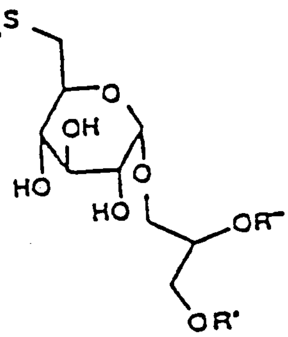

worin R' ein geradkettiger gesättigter oder ungesättigter Fettsäureester und R" ein geradkettiger gesättigter Fettsäureester ist, bei der Herstellung eines Arzneimittels für die Behandlung von viralen Infektionen eines Wirts.

9. Verwendung gemäß Anspruch 8, worin besagter R"-Fettsäureester ein Kohlenstoffatom zu Doppelbindung-Verhältnis von 16:0 hat und der R'-Fettsäureester ein Kohlenstoffatom zu Doppelbindung-Verhältnis, ausgewählt aus der Gruppe bestehend aus 18:3, 18:2, 18:1 und 16:1, hat.

10. Verwendung gemäß Anspruch 8, worin besagte virale Infektion durch Human-Immunmangel-Virus verursacht ist.

11. Verwendung gemäß Anspruch 9, worin besagte Sulfoquinovosylglycerol-Verbindung eine Verbindung der folgenden Formel ist:

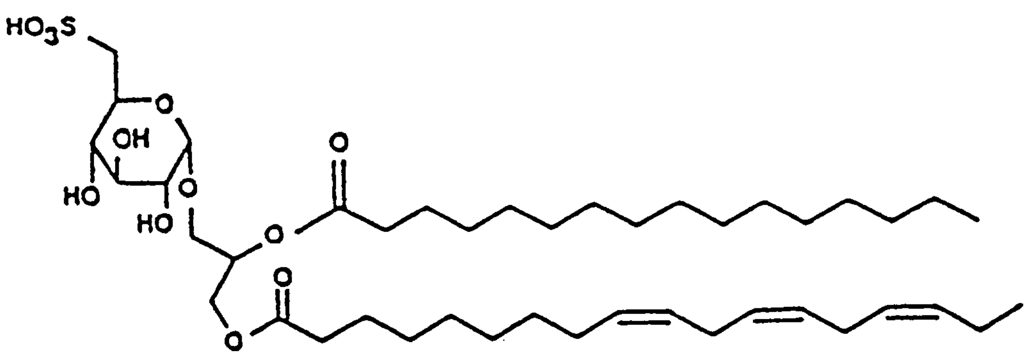

12. Verwendung gemäß Anspruch 9, worin R' 18:2 ist.

13. Verwendung gemaß Anspruch 9, worin R' 18:1 ist.

14. Verwendung gemäß Anspruch 9, worin R' 16:1 ist.

15. Verwendung gemäß Anspruch 9, worin R' 18:0 ist.

## Revendications

**1.** Composition comprenant un dérivé de sulfoquinovosyl

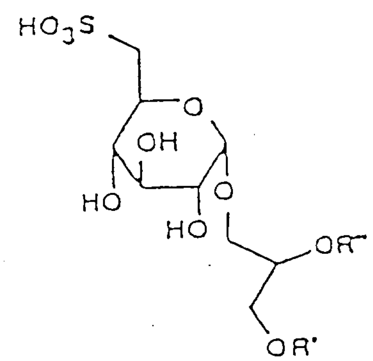

glycérol de formule : dans laquelle R' représente un ester d'acide gras saturé ou insaturé à chaîne linéaire, et R'' représente un ester d'acide gras saturé à chaîne linéaire ; et un excipient pharmaceutiquement acceptable ; destinée à être employée pour le traitement d'infections virales.

**2.** Composition pour l'utilisation selon la revendication 1, dans laquelle l'ester d'acide gras R'' a un rapport des nombres d'atomes de carbone et de doubles liaisons, de 16:0, et l'ester d'acide gras R' a un rapport des nombres d'atomes de carbone et de doubles liaison choisi parmi 18:3, 18:2, 18:1 et 16:1.

**3.** Composition pour l'utilisation selon la revendication 2, dans laquelle le composé dérivé de sulfoquinovosyl glycérol, est un composé correspondant à la formule suivante :

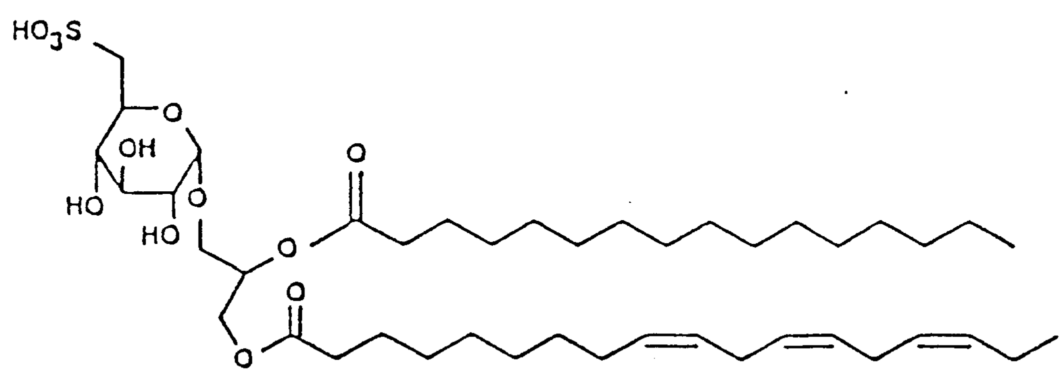

**4.** Composition pour l'utilisation selon la revendication 2, dans laquelle R' correspond à 18:2.

**5.** Composition pour l'utilisation selon la revendication 2, dans laquelle R' correspond à 18:1.

**6.** Composition pour l'utilisation selon la revendication 2, dans laquelle R' correspond à 16:1.

**7.** Composition pour l'utilisation selon la revendication 2, dans laquelle R' correspond à 18:0.

8. Utilisation d'un composé dérivé de sulfoquinovosyl glycérol de formule :

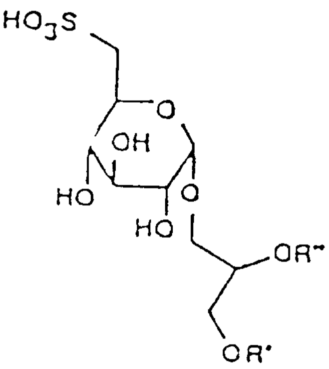

dans laquelle R' représente un ester d'acide gras saturé ou insaturé à chaîne linéaire, et R'' représente un ester d'acide gras saturé à chaîne linéaire, pour la préparation d'un médicament pour le traitement d'infections virales d'un hôte.

9. Utilisation selon la revendication 8, dans laquelle l'ester d'acide gras R'', a un rapport des nombres d'atomes de carbone et de doubles liaisons de 16:0, et l'ester d'acide gras R' a un rapport des nombres d'atomes de carbone et de doubles liaisons, choisi parmi 18:3, 18:2, 18:1 et 16:1.

10. Utilisation selon la revendication 8, dans laquelle l'infection virale est due au virus de l'immunodéficience humaine.

11. Utilisation selon la revendication 9, dans laquelle le composé dérivé de sulfoquinovosyl glycérol, est un composé correspond à la formule suivante :

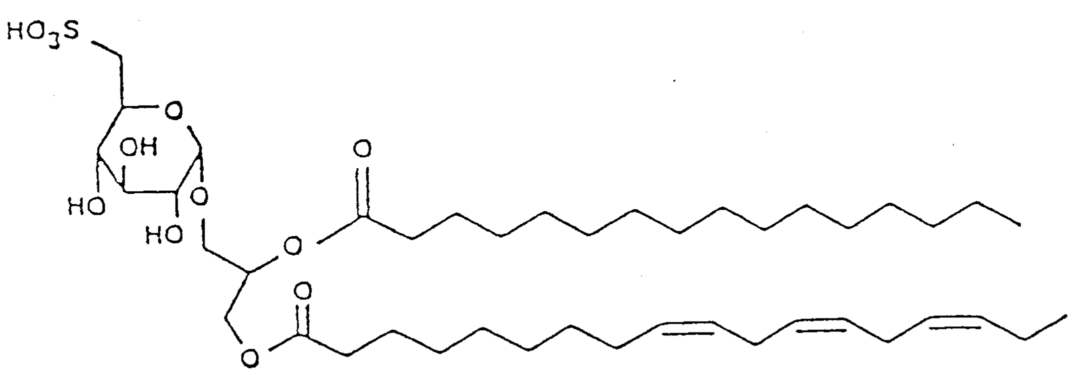

12. Utilisation selon la revendication 9, dans laquelle R' correspond à 18:2.

13. Utilisation selon la revendication 9, dans laquelle R' correspond à 18:1.

14. Utilisation selon la revendication 9, dans laquelle R' correspond à 16:1.

15. Utilisation selon la revendication 9, dans laquelle R' correspond à 18:0.

FIG. 1A

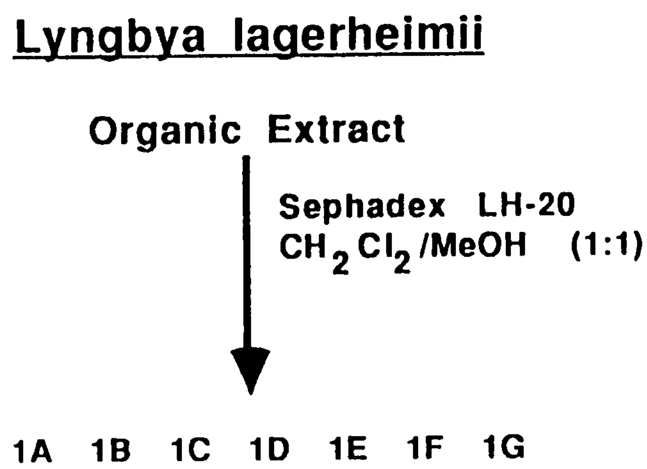
Lyngbya lagerheimii
Organic Extract
Sephadex LH-20
$CH_2Cl_2$/MeOH (1:1)
1A 1B 1C 1D 1E 1F 1G

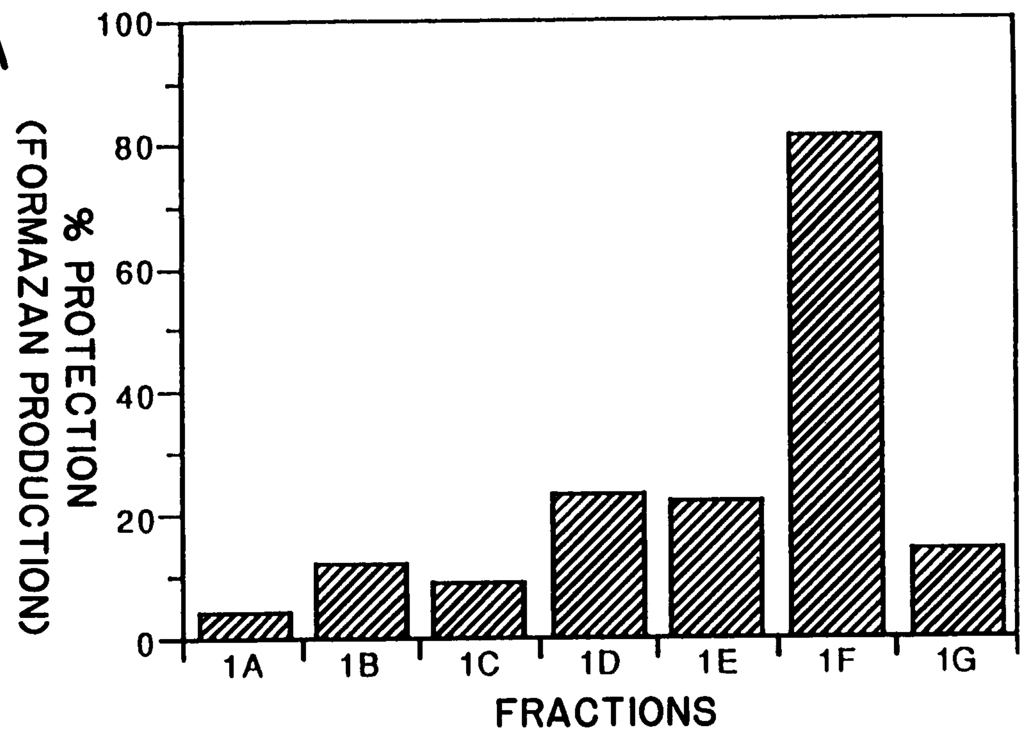
% PROTECTION
(FORMAZAN PRODUCTION)
0
20
40
60
80
100
1A
1B
1C
1D
1E
1F
1G
FRACTIONS

FIG. 1B

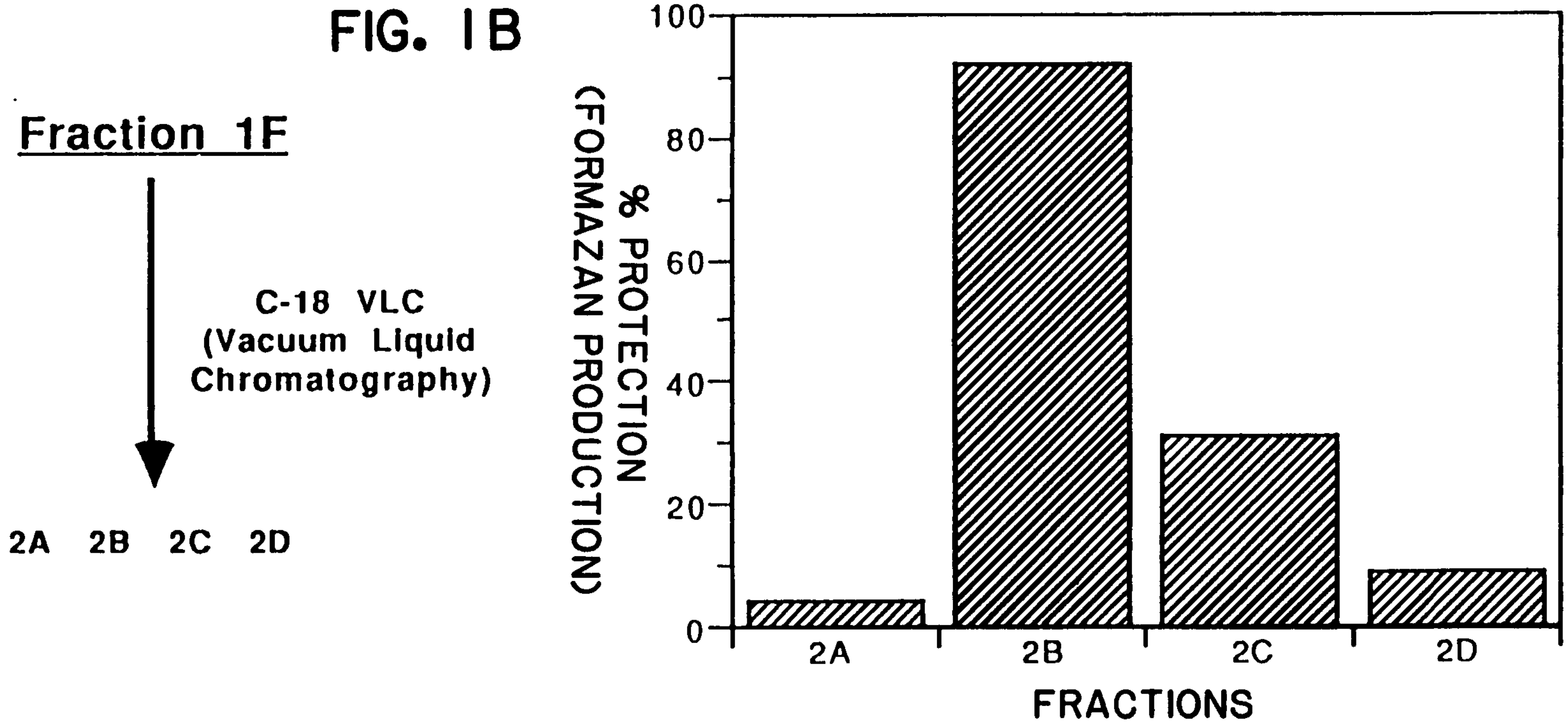
Fraction 1F
C-18 VLC
(Vacuum Liquid
Chromatography)
2A 2B 2C 2D
% PROTECTION
(FORMAZAN PRODUCTION)
0
20
40
60
80
100
2A
2B
2C
2D
FRACTIONS

FIG. 1C

**Fraction 2B**

C-18 HPLC
MeOH/$H_2O$ (9:1)

Pure Sulfolipids

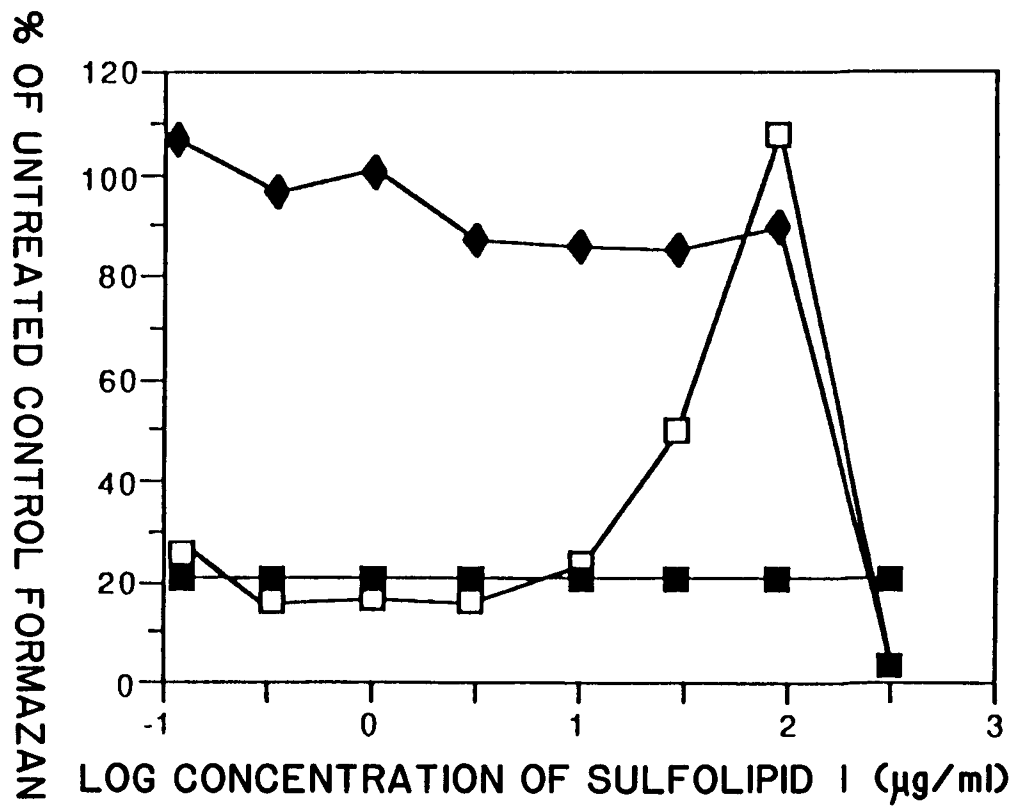

FIG. 2A

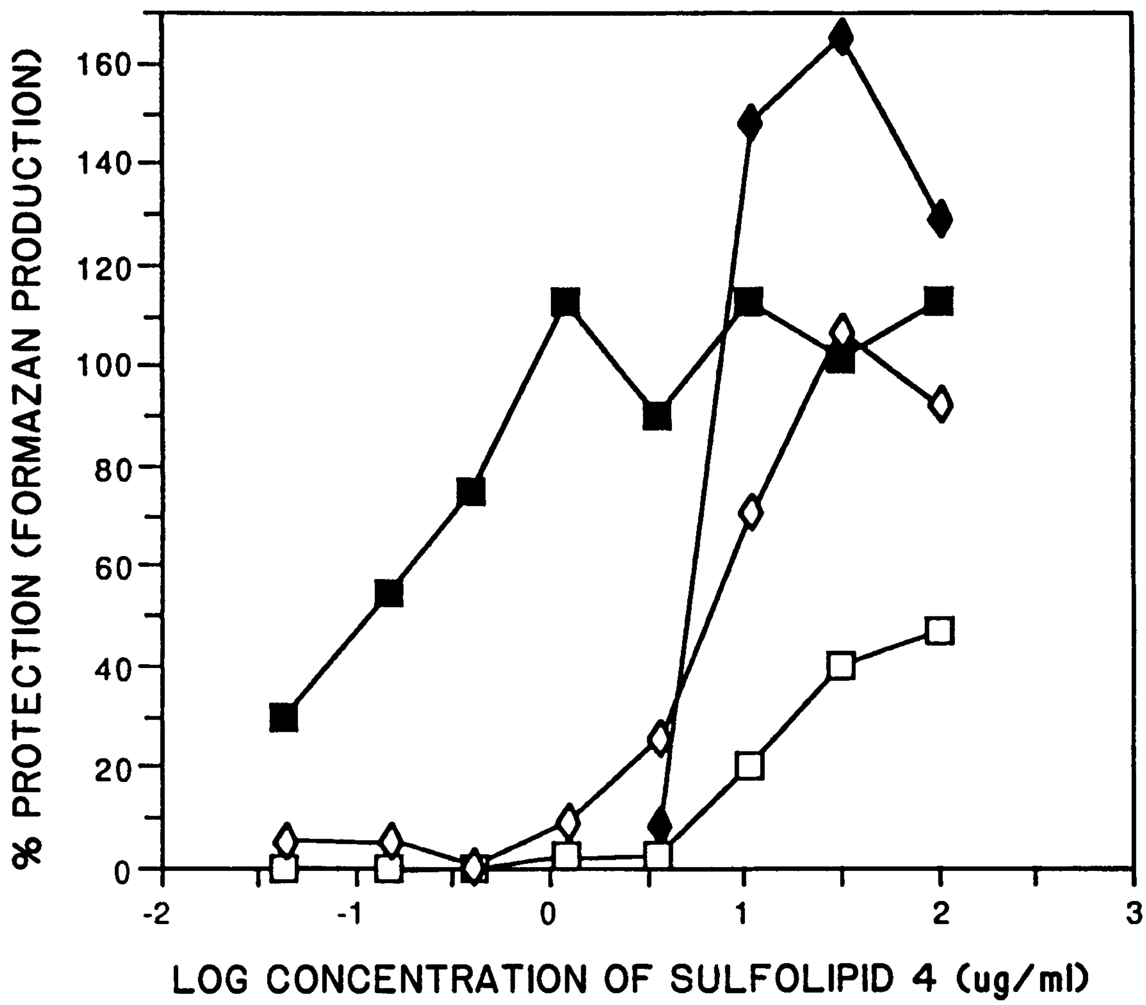
% PROTECTION (FORMAZAN PRODUCTION)
0
20
40
60
80
100
120
140
160
-2
-1
0
1
2
3
LOG CONCENTRATION OF SULFOLIPID 4 (ug/ml)

FIG. 2B

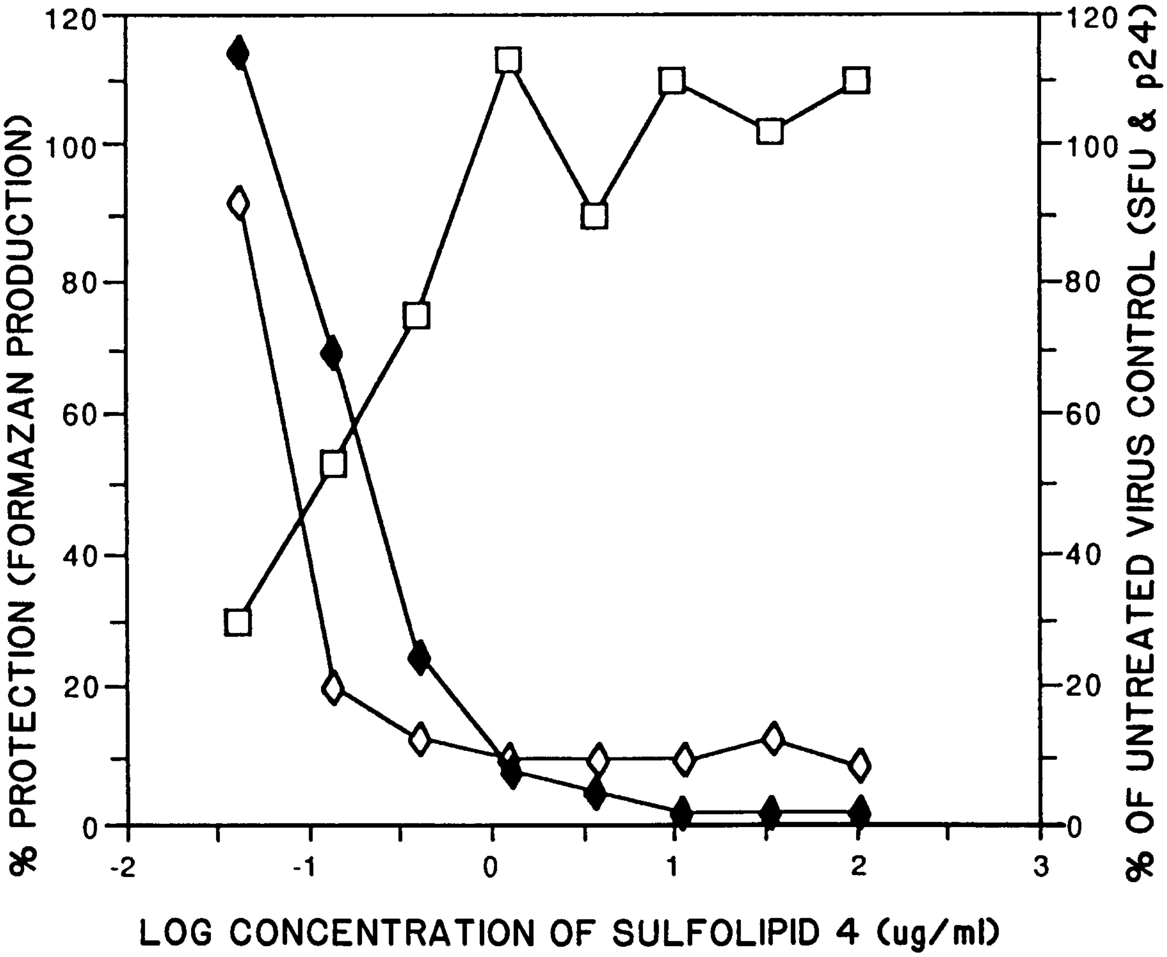
120
100
80
60
40
20
0
% PROTECTION (FORMAZAN PRODUCTION)
120
100
80
60
40
20
0
% OF UNTREATED VIRUS CONTROL (SFU & p24)
-2
-1
0
1
2
3
LOG CONCENTRATION OF SULFOLIPID 4 (ug/ml)

FIG. 3